# EUROPEAN PATENT SPECIFICATION

(11) **EP 3 809 125 B1**
(45) Date of publication and mention of the grant of the patent: **04.09.2024**
(21) Application number: 19823522.8
(22) Date of filing: 10.06.2019
(51) Int. Cl.: G01N 27/04, A61B 5/00, G01N 33/497

(54) **BREATHALYZER CAPABLE OF ESTIMATING REMAINING LIFE OF DETECTION UNIT**
ATEMALKOHOLMESSGERÄT MIT SCHÄTZUNG DER RESTLEBENSDAUER DER DETEKTIONSEINHEIT
ÉTHYLOMÈTRE CAPABLE D'ESTIMER LA DURÉE DE VIE RESTANTE D'UNE UNITÉ DE DÉTECTION

(30) Priority: 18.06.2018 KR 20180069438
(43) Date of publication of application: 21.04.2021
(73) Proprietor: Sentech Korea Corp., Paju-si, Gyeonggi-do 10880 (KR)
(72) Inventor: YOO, Do Joon, Seongnam-si, Gyeonggi-do 13456 (KR); KIM, Soo Hyun, Goyang-si, Gyeonggi-do 10412 (KR)
(74) Representative: v. Bezold & Partner Patentanwälte - PartG mbB
(86) International application number: PCT/KR2019/006933
(87) International publication number: WO 2019/245206

(56) References cited:
- GB-A- 2 201 245
- JP-A- H0 755 761
- JP-B2- 6 229 836
- JP-B2- 6 229 836
- KR-A- 20030 017 878
- KR-A- 20030 017 878
- KR-A- 20140 022 638
- KR-A- 20140 022 638
- KR-A- 20140 024 993
- KR-A- 20180 064 478
- US-A1- 2017 303 819

## Description

### Technical Field

The present invention relates to a breathalyzer and, more particularly, to a breathalyzer capable of estimating the remaining life of a sensing unit.

### Background Art

A breathalyzer measures the concentration of alcohol mixed with the air of inhalation and discharged together with exhalation. Since the alcohol discharged with exhalation is proportional to the concentration of alcohol in the blood, blood alcohol concentration (BAC) can be calculated by measuring the concentration of alcohol in the exhalation.

The breathalyzer includes an exhalation passage through which exhalation flows, a sample gas chamber which communicates with the exhalation passage so that a part of the exhalation flows into the exhalation passage, and a sensing unit disposed inside the sample gas chamber to generate an electric signal according to the concentration of alcohol contained in the exhalation.

The alcohol sensor of the sensing unit is divided into an electrochemical alcohol sensor and a semiconductor alcohol sensor. The semiconductor alcohol sensor uses a change in the electrical conductivity of a ceramic semiconductor when a gas makes contact with the surface of a ceramic semiconductor such as SnO₂, In₂O₃, ZnO or the like. The semiconductor alcohol sensor has a long lifespan, a high response speed, and relatively low accuracy.

The electrochemical alcohol sensor uses a current flowing between a reaction electrode and a counterpart electrode due to the oxidation/reduction reaction occurring in the reaction electrode and the counterpart electrode. The electrochemical alcohol sensor has high accuracy, a short lifespan and a low response speed.

The electrochemical alcohol sensor includes a reaction electrode in which the oxidation/reduction reaction of alcohol occurs, a counterpart electrode, a liquid electrolyte interposed between the reaction electrode and the counterpart electrode while being carried on a porous carrier, and a platinum lead wire bonded to the reaction electrode and the counterpart electrode.

The electrochemical alcohol sensor has a problem that when the liquid electrolyte evaporates, the output current value is changed according to the alcohol concentration, which makes it difficult to perform accurate measurement. Since the degree of evaporation of the liquid electrolyte in the electrochemical alcohol sensor is greatly affected by the use environment of the breathalyzer, especially the temperature, it is difficult to predict the remaining life of the alcohol sensor. For example, when a breathalyzer is left inside a vehicle in summer, the liquid electrolyte in the alcohol sensor evaporates within about 12 hours, which may make it difficult to perform accurate measurement.

### [Prior Art Documents]

### [Patent documents]

US 2017/303819 discloses a breathalyser comprising a remaining life estimation unit configured to estimate the remaining life of the sensing unit by comparing a time value detected by the time detection unit with a reference value.

Korean Patent No. 10-0489302 Korean Patent Application Publication No. 10-2008-0076515 Korean Utility Model Application Publication No. 97-59155 Document JP 6 229836 B2 discloses an alcohol sensor that outputs an electric signal according to the concentration of alcohol contained in the expiration of a subject. An exposing mechanism exposes the alcohol sensor to external air when measuring the concentration of alcohol contained in the expiration of the subject. A CPU measures the concentration of alcohol contained in the expiration of the subject on the basis of the electric signal output by the alcohol sensor when the alcohol sensor is exposed to the external air. The CPU displays a notification on replacement of the alcohol sensor on a display when the total sum of values corresponding to the lengths of time of exposure of the alcohol sensor to the external air has exceeded a prescribed level.

### Summary

The present invention provides a breathalyzer according to independent claim 1, capable of minimizing the occurrence of an error due to the evaporation of a liquid electrolyte by estimating the remaining life of a sensing unit and allowing a user to replace the sensing unit before the end of its life.

In order to achieve the above object, the present invention provides a breathalyzer, including: an exhalation passage through which exhalation flows; a sample gas chamber communicating with the exhalation passage; a sensing unit disposed inside the sample gas chamber; and a controller, wherein the controller includes a sensing signal receiving unit configured to receive an electric signal changed over time from the sensing unit, a time detection unit configured to detect time-related information from the electric signal received by the sensing signal receiving unit, and a remaining life estimation unit configured to estimate the remaining life of the sensing unit by comparing a time value detected by the time detection unit with a reference value.

In the breathalyzer, the time detection unit may be configured to detect a time value corresponding to a maximum value of the electric signal received by the sensing signal receiving unit.

In the breathalyzer, the reference value may be a time value obtained by using a standard gas in a calibration step and corresponding to the maximum value of the electric signal received by the sensing signal receiving unit.

In the breathalyzer, the time detection unit may be configured to detect a time value taken until the electric signal received by the sensing signal receiving unit decreases past a maximum value and reaches a value obtained by multiplying the maximum value by a predetermined ratio.

In the breathalyzer, the reference value may be a time value obtained by using a standard gas in a calibration step and taken until the electric signal received by the sensing signal receiving unit decreases past a maximum value and reaches a value obtained by multiplying the maximum value by the predetermined ratio.

In the breathalyzer, the predetermined ratio may be a value selected from 2% to 20%.

The breathalyzer may further include: a temperature sensor configured to measure a temperature value, wherein the controller may be configured to estimate the remaining life of the sensing unit only when the temperature value measured by the temperature sensor is 20 to 28 degrees C.

In the breathalyzer, the controller includes a blood alcohol concentration calculation unit configured to calculate a blood alcohol concentration based on an electrical signal, preferably by comparing a value obtained by integrating the current value, received from the sensing unit, preferably over time with a reference integral value. According to the invention, the remaining life estimation unit is configured to estimate the remaining life of the sensing unit only when the blood alcohol concentration measured by the blood alcohol concentration calculation unit is 0.02BAC% or higher.

Since the breathalyzer according to the present invention can estimate the remaining life of the sensing unit by analyzing the output data of the sensing unit, the remaining life of the sensing unit can be notified to the user in advance before the end of the life of the sensing unit. Therefore, it is possible to prevent the occurrence of a measurement error due to the use of the sensing unit that has reached the end of its life.

### Brief Description of the Drawings

FIG. 1 is a conceptual diagram schematically showing a breathalyzer according to the present invention.
FIG. 2 is a view illustrating an output signal of a sensing unit of the breathalyzer shown in FIG. 1.
FIG. 3 is a view showing a change in an output signal according to the remaining life of the sensing unit of the breathalyzer shown in FIG. 1.
FIG. 4 is a block diagram of the controller shown in FIG. 1.

### Detailed Description

Hereinafter, a preferred embodiment of a breathalyzer according to the present invention will be described in detail with reference to the accompanying drawings.

The following embodiments are provided by way of example in order to sufficiently convey the scope of the present invention to those skilled in the art. Therefore, the present invention is not limited to the embodiments described below and may be embodied in other forms. In the drawings, the width, length, thickness and the like of each component may be exaggerated for the sake of convenience. Like reference numerals indicate the same elements throughout the specification.

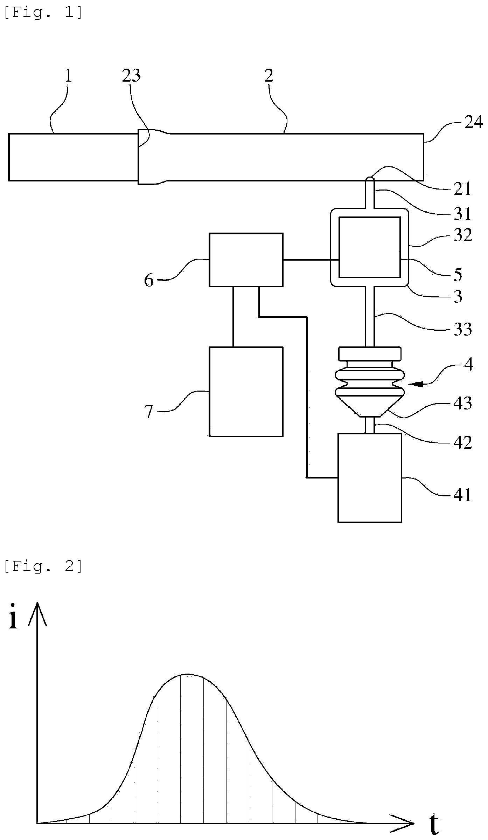

FIG. 1 is a conceptual diagram schematically showing a breathalyzer according to the present invention. Referring to FIG. 1, the breathalyzer according to the present invention includes an exhalation passage 2, a sample gas chamber 3 communicating with the exhalation passage 2, and a suction pump 4 connected to the sample gas chamber 3.

The exhalation passage 2 is in the form of a cylinder having opposite open ends. A blow tube 1 may be coupled to the inlet 23 at one end of the exhalation passage 2. The exhalation introduced into exhalation passage 2 through the blow tube 1 is discharged to the outside through the outlet 24 at the other end of the exhalation passage 2.

The exhalation passage 2 is formed with a sampling hole 21 for supplying a part of the exhalation introduced into the exhalation passage 2 to the sample gas chamber 3. Most of the exhalation introduced into the exhalation passage 2 is immediately discharged to the outside through the outlet 24, and a part of the exhalation is supplied to the sample gas chamber 3 through the sampling hole 21. Hereinafter, the exhalation flowing through the exhalation passage 2 and supplied through the sampling hole 21 will be referred to as a sample gas.

The sample gas chamber 3 is connected to the sampling hole 21 of the exhalation passage 2 through a sample gas inlet pipe 31. A sensing unit 5 is installed inside the sample gas chamber 3. The sensing unit 5 outputs a current value changed according to the alcohol concentration in the sample gas. As the sensing unit 5, an electrochemical reaction cell may be used.

The electrochemical reaction cell includes a sensing electrode, a counterpart electrode and an electrolyte interposed between the sensing electrode and the counterpart electrode. Alcohol adsorbed to the sensing electrode and decomposed into ions passes through the electrolyte and then moves to the counterpart electrode. The current value obtained at this time is changed over time as shown in FIG. 2. By integrating the graph of change of current value over time, it is possible to obtain a sensing signal value proportional to the amount of alcohol adsorbed to the sensing electrode and decomposed. A blood alcohol concentration can be determined using the sensing signal value. The integral value is proportional to the amount of alcohol adsorbed to the sensing electrode, the amount of alcohol adsorbed is proportional to the alcohol concentration in the sample gas, and the alcohol concentration in the sample gas is proportional to the blood alcohol concentration.

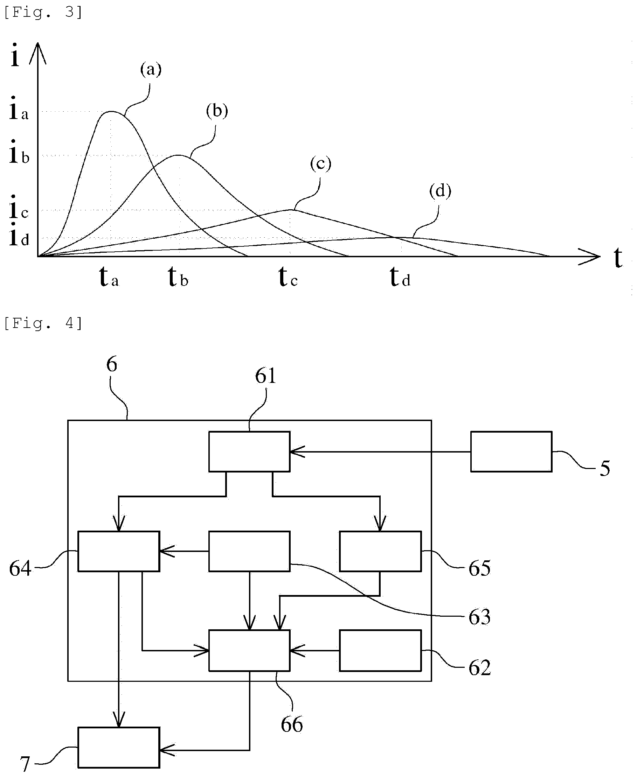

FIG. 3 is a view showing a change in the output signal according to the remaining life of the sensing unit of the breathalyzer shown in FIG. 1. The remaining life decreases from (a) to (d) in FIG. 3. As shown in Fig. 3, the total measurement time becomes longer as the remaining life decreases (the use time increases). In addition, as the remaining life of the sensing unit decreases, the time taken until the maximum value of the graph of change of the current value of the sensing unit appears becomes longer from tₐ to t_{d}. This is because the reaction rate drops due to the evaporation of the electrolyte. The total measurement time and the time taken until the maximum value of the current value appears are not significantly affected by the alcohol concentration.

Even if the reaction rate drops due to the evaporation of the electrolyte, the magnitude of the value obtained by integrating the graph of change of the current value over time is maintained to a certain extent. For example, if the alcohol concentration in the sample gas is the same, the values obtained by integrating (a) and (b) of FIG. 3 over time may be substantially the same. However, if the electrolyte evaporates too much, the adsorbed alcohol is not completely decomposed. Thus, the magnitude of the value obtained by integrating the graph of change of the current value begins to decrease. For example, even if the alcohol concentration in the sample gas is the same, the value obtained by integrating (c) and (d) of FIG. 3 over time may be smaller than the value obtained by integrating (a) of FIG. 3 over time. If the magnitude of the value obtained by integrating the graph of change of the current value decreases, the exact blood alcohol concentration value cannot be measured. Therefore, the sensing unit 5 needs to be replaced. In the present invention, the remaining life is estimated by taking advantage of the fact that as the remaining life decreases, the total measurement time increases and the time taken until the maximum value of the graph of change of the current value appears increases.

The suction pump 4 serves to introduce a predetermined amount of the sample gas blown by the user through the blow tube 1 into the sample gas chamber 3 and discharge the sample gas from the sample gas chamber 3. The suction pump 4 is connected to the sample gas chamber 3 through a connection pipe 34.

Since the suction pump 4 has a closed structure, when the suction pump 4 is not operated, almost no gas flows into the sample gas chamber 3.

The suction pump 4 is operated instantaneously after a certain period of time has elapsed after the user starts to exhale, thereby introducing a predetermined amount of the sample gas into the sample gas chamber 3. Then, the introduced sample gas is discharged from the sample gas chamber 3 through the sample gas inlet pipe 31. Whether the user starts to exhale can be checked by using, for example, a microphone (not shown) or a pressure sensor (not shown) installed in the exhalation passage 2.

A solenoid pump may be used as the suction pump 4. The solenoid pump includes a housing 41 in which a bobbin (not shown) wound with an induction coil is installed, and a plunger 42 installed to move up and down inside the housing 41. The plunger 42 is connected to a bellows 43 that can be expanded and contracted.

When electric power is momentarily applied to the induction coil, the plunger 42 retreats by a certain distance and then immediately returns by a return spring (not shown) in the housing 41. In the retreating process of the plunger 42, the bellows 43 expands and the sample gas flows into the sample gas chamber 2. In the returning process of the plunger 42, the bellows 43 contracts and the sample gas in the sample gas chamber 32 is discharged through the sample gas inlet pipe 31. By limiting the moving distance of the plunger 42, a predetermined amount of the sample gas can be sampled and a predetermined of the sample gas can be discharged. The sample gas is adsorbed to the electrochemical reaction cell of the sensing unit 5 disposed in the sample gas chamber 3 when the sample gas is introduced into and discharged from the sample gas chamber 3.

In addition, the breathalyzer may further include a temperature sensor (not shown). The temperature sensor generates an electric signal according to the ambient temperature during a sobriety test.

The controller 6 serves to transmit a control signal for operating the suction pump 4 to the suction pump 4. In addition, the controller 6 functions to calculate a blood alcohol concentration value according to the electric signal value received from the sensing unit 5. Further, the controller 6 serves to estimate the remaining life of the sensing unit 5 according to the electric signal value received from the sensing unit 5.

As shown in FIG. 4, the controller 6 includes a sensing signal receiving unit 61, a temperature signal receiving unit 62, a memory 63, a blood alcohol concentration calculating unit 64, a time detection unit 65 and a remaining life estimation unit 66.

The sensing signal receiving unit 61 serves to receive an electric signal changed over time from the sensing unit 5.

The temperature signal receiving unit 62 serves to receive an electric signal according to the ambient temperature from a temperature sensor.

The memory 63 stores a reference value used to estimate the remaining life. The reference value is time-related information detected from the electric signal received by the sensing signal receiving unit 61 when a standard sample gas is allowed to flow into the breathalyzer in a step of calibrating the breathalyzer equipped with a new sensing unit 5 at a room temperature. For example, the reference value may be the time at which the electric signal received by the sensing signal receiving unit 61 indicates a maximum value or the total analysis time. The total analysis time may be the time taken until the electric signal received by the sensing signal receiving unit 61 decreases past the maximum value and reaches a value obtained by multiplying the maximum value by a predetermined ratio. The predetermined ratio may be a value selected from 2% to 20%.

Further, the memory 63 also stores a reference integral value, which is an output signal of the sensing unit 5 obtained by using a standard gas.

The blood alcohol concentration calculation unit 64 calculates a blood alcohol concentration by comparing a value obtained by integrating the current value received from the sensing unit 5 over time with the reference integral value stored in the memory 63.

For example, if the reference integral value obtained by using the standard gas corresponding to 0.05BAC% is "a" and the measured integral value is 0.5a, the blood alcohol concentration is calculated as 0.025BAC%, which is 0.5×0.05BAC%.

The time detection unit 65 detects time-related information from the electric signal received by the sensing signal receiving unit 61.

For example, the time detection unit 65 may detect the total analysis time. The total analysis time may be detected by measuring a time value taken until the electric signal received by the sensing signal receiving unit 61 decreases past the maximum value and reaches a value obtained by multiplying the maximum value by a predetermined ratio. Since there is noise in the electric signal, it is not possible to measure the time value taken until the electric signal becomes completely zero. Therefore, the time taken until the electric signal reaches the time obtained by multiplying the maximum value by a predetermined ratio is measured instead thereof. The predetermined ratio may be a value selected from 2% to 20%.

In addition, the time detection unit 65 may detect a time value corresponding to the maximum value of the electric signal received by the sensing signal receiving unit 61. For example, the time detection unit 62 may differentiate the electric signal received by the sensing signal receiving unit 61 according to time, and may extract the time at which the differential value becomes 0 as a time value corresponding to the maximum value of the electric signal. In addition, the time point at which the electric signal received by the sensing signal receiving unit 61 decreases may be extracted as a time value corresponding to the maximum value.

The remaining life estimation unit 66 serves to estimate the remaining life of the sensing unit 5 by comparing the time value detected by the time detection unit 65 with the reference value stored in the memory 63 when the blood alcohol concentration calculated by the blood alcohol concentration unit is 0.02BAC% or higher. The remaining life estimation unit 66 may estimate the remaining life using a ratio of the detected time value and the reference value. For example, if the detected time value is 3 times or more of the reference value, it may be determined that the sensing unit 5 needs to be replaced.

Before estimating the remaining life, the remaining life estimation unit 66 first checks whether the remaining life can be estimated. That is, the remaining life estimation unit 66 first calculates an ambient temperature value at the time of measurement by using the electric signal received from the temperature signal receiving unit 62 and determines whether the temperature value falls within a range of 20 to 28 degrees C. This is because if the measurement is performed at a low temperature of less than 20 degrees C, the total measurement time and the time required for reaching the maximum value increase due to the influence of the ambient temperature, as a result of which the end of the life of the sensing unit 5 may be erroneously measured.

In addition, the remaining life estimation unit 66 determines whether the blood alcohol concentration measured by the blood alcohol concentration calculation unit 64 is 0.02BAC% or more. This is because it is difficult to accurately estimate the remaining life when the alcohol concentration is too low.

The blood alcohol concentration value calculated by the blood alcohol concentration calculation unit 64 is displayed on the display unit 7. In addition, when the remaining life estimation unit 66 determines that the remaining life of the sensing unit 5 is short, a warning indicating that the replacement of the sensing unit 5 is necessary may be displayed on the display unit 7.

While the preferred embodiment of the present invention has been shown and described above, the present invention is not limited to the specific embodiment described above.

### [Description of Reference Numerals]

1: blow tube, 2: exhalation passage, 3: sample gas chamber, 31: sample gas inlet pipe, 34: connection pipe, 4: suction pump, 5: sensing unit, 6: controller, 7: display unit

## Claims

1. A breathalyzer, comprising:
an exhalation passage (2) through which exhalation flows;
a sample gas chamber (3) communicating with the exhalation passage;
a sensing unit (5) disposed inside the sample gas chamber; and
a controller (6)
wherein the controller includes a sensing signal receiving unit (61) configured to receive an electric signal changed over time from the sensing unit, a time detection unit (65) configured to detect time-related information from the electric signal received by the sensing signal receiving unit, and a blood alcohol concentration calculation unit (64) configured to calculate a blood alcohol concentration based on an electric signal from the sensing unit, **characterized in that**
the controller includes a remaining life estimation unit (66) configured to estimate the remaining life of the sensing unit by comparing a time value detected by the time detection unit with a reference value only when the blood alcohol concentration calculated by the blood alcohol concentration unit is 0.02BAC% or higher.

2. The breathalyzer according to claim 1, wherein the time detection unit is configured to detect a time value corresponding to a maximum value of the electric signal received by the sensing signal receiving unit.

3. The breathalyzer according to claim 2, wherein the reference value is a time value obtained by using a standard gas in a calibration step and corresponding to the maximum value of the electric signal received by the sensing signal receiving unit.

4. The breathalyzer according to claim 1, wherein the time detection unit is configured to detect a time value taken until the electric signal received by the sensing signal receiving unit decreases past a maximum value and reaches a value obtained by multiplying the maximum value by a predetermined ratio.

5. The breathalyzer according to claim 4, wherein the reference value is a time value obtained by using a standard gas in a calibration step and taken until the electric signal received by the sensing signal receiving unit decreases past a maximum value and reaches a value obtained by multiplying the maximum value by the predetermined ratio.

6. The breathalyzer according to claim 4, wherein the predetermined ratio is a value selected from 2% to 20%.

7. The breathalyzer according to claim 1, further comprising:
a temperature sensor configured to measure a temperature value,
wherein the controller is configured to estimate the remaining life of the sensing unit only when the temperature value measured by the temperature sensor is 20 to 28 degrees C.

8. The breathalyzer according to claim 1, wherein the blood alcohol concentration calculation unit is configured to calculate the blood alcohol concentration by comparing a value obtained by integrating the current value received from the sensing unit over time with a reference integral value.

## Patentansprüche

1. Atemmessvorrichtung, umfassend:
einen Exhalationskanal (2), durch den eine Exhalation strömt;
eine Probengaskammer (3), die mit dem Exhalationskanal in Verbindung steht;
eine Erfassungseinheit (5), die innerhalb der Probengaskammer angeordnet ist; und
eine Steuereinrichtung (6), wobei die Steuereinrichtung eine Erfassungssignal-Empfangseinheit (61) umfasst, die konfiguriert ist, ein über die Zeit verändertes elektrisches Signal von der Erfassungseinheit zu empfangen, eine Zeiterfassungseinheit (65), die konfiguriert ist, zeitbezogene Informationen aus dem von der Erfassungssignal-Empfangseinheit empfangenen elektrischen Signal zu erfassen, und eine Blutalkoholkonzentrations-Berechnungseinheit (64), die konfiguriert ist, eine Blutalkoholkonzentration auf der Basis eines elektrischen Signals von der Erfassungseinheit zu berechnen, **dadurch gekennzeichnet, dass**
die Steuereinrichtung eine Einheit (66) zur Abschätzung der verbleibenden Lebensdauer umfasst, die konfiguriert ist, die verbleibende Lebensdauer der Erfassungseinheit abzuschätzen, indem ein von der Zeiterfassungseinheit erfasster Zeitwert nur dann mit einem Referenzwert verglichen wird, wenn die von der Blutalkoholkonzentrations-Einheit berechnete Blutalkoholkonzentration 0,02BAC% oder höher ist.

2. Atemmessvorrichtung nach Anspruch 1, wobei die Zeiterfassungseinheit konfiguriert ist, einen Zeitwert zu erfassen, der einem Maximalwert des elektrischen Signals entspricht, das von der Erfassungssignal-Empfangseinheit empfangen wird.

3. Atemmessvorrichtung nach Anspruch 2, wobei der Referenzwert ein Zeitwert ist, der durch Verwendung eines Standardgases in einem Kalibrierungsschritt erhalten wird und dem Maximalwert des von der Erfassungssignal-Empfangseinheit empfangenen elektrischen Signals entspricht.

4. Atemmessvorrichtung nach Anspruch 1, wobei die Zeiterfassungseinheit konfiguriert ist, einen Zeitwert zu erfassen, der genommen wird, bis das von der Erfassungssignal-Empfangseinheit empfangene elektrische Signal über einen Maximalwert hinaus abnimmt und einen Wert erreicht, der durch Multiplizieren des Maximalwerts mit einem vorbestimmten Verhältnis erhalten wird.

5. Atemmessvorrichtung nach Anspruch 4, wobei der Referenzwert ein Zeitwert ist, der durch Verwendung eines Standardgases in einem Kalibrierungsschritt erhalten wird und genommen wird, bis das von der Erfassungssignal-Empfangseinheit empfangene elektrische Signal über einen Maximalwert hinaus abnimmt und einen Wert erreicht, der durch Multiplikation des Maximalwertes mit dem vorbestimmten Verhältnis erhalten wird.

6. Atemmessvorrichtung nach Anspruch 4, wobei das vorbestimmte Verhältnis ein Wert ist, der aus 2 % bis 20 % ausgewählt ist.

7. Atemmessvorrichtung nach Anspruch 1, ferner umfassend:
einen Temperatursensor, der konfiguriert ist, einen Temperaturwert zu messen,
wobei die Steuereinrichtung konfiguriert ist, die verbleibende Lebensdauer der Erfassungseinheit nur dann abzuschätzen, wenn der von dem Temperatursensor gemessene Temperaturwert 20 bis 28 Grad C beträgt.

8. Atemmessvorrichtung nach Anspruch 1, wobei die Blutalkoholkonzentrations-Berechnungseinheit konfiguriert ist, die Blutalkoholkonzentration durch Vergleichen eines Wertes, der durch Integrieren des von der Erfassungseinheit empfangenen aktuellen Wertes über die Zeit erhalten wird, mit einem Referenz-Integralwert zu berechnen.

## Revendications

1. Ethylomètre, comprenant :
un passage d'expiration (2) à travers lequel circule l'expiration ;
une chambre de gaz échantillon (3) communiquant avec le passage d'expiration ;
une unité de détection (5) disposée à l'intérieur de la chambre de gaz échantillon ;
et
un dispositif de commande (6)
dans lequel le dispositif de commande comporte une unité de réception de signal de détection (61) configurée pour recevoir de l'unité de détection un signal électrique ayant changé au fil du temps, une unité de détection de temps (65) configurée pour détecter des informations liées au temps à partir du signal électrique reçu par l'unité de réception de signal de détection, et une unité de calcul d'alcoolémie (64) configurée pour calculer une alcoolémie sur la base d'un signal électrique provenant de l'unité de détection, **caractérisé en ce que**
le dispositif de commande comporte une unité d'estimation de durée de vie restante (66) configurée pour estimer la durée de vie restante de l'unité de détection en comparant une valeur de temps détectée par l'unité de détection de temps à une valeur de référence uniquement lorsque l'alcoolémie calculée par l'unité d'alcoolémie est supérieure ou égale à 0,02 BAC%.

2. Ethylomètre selon la revendication 1, dans lequel l'unité de détection de temps est configurée pour détecter une valeur de temps correspondant à une valeur maximale du signal électrique reçu par l'unité de réception de signal de détection.

3. Ethylomètre selon la revendication 2, dans lequel la valeur de référence est une valeur de temps obtenue au moyen d'un gaz standard dans une étape d'étalonnage et correspondant à la valeur maximale du signal électrique reçu par l'unité de réception de signal de détection.

4. Ethylomètre selon la revendication 1, dans lequel l'unité de détection de temps est configurée pour détecter une valeur de temps prise jusqu'à ce que le signal électrique reçu par l'unité de réception de signal de détection diminue au-delà d'une valeur maximale et atteigne une valeur obtenue par multiplication de la valeur maximale par un taux prédéterminé.

5. Ethylomètre selon la revendication 4, dans lequel la valeur de référence est une valeur de temps obtenue au moyen d'un gaz standard dans une étape d'étalonnage et prise jusqu'à ce que le signal électrique reçu par l'unité de réception de signal de détection diminue au-delà d'une valeur maximale et atteigne une valeur obtenue par multiplication de la valeur maximale par le taux prédéterminé.

6. Ethylomètre selon la revendication 4, dans lequel le taux prédéterminé est une valeur sélectionnée de 2% à 20%.

7. Ethylomètre selon la revendication 1, comprenant en outre :
un capteur de température configuré pour mesurer une valeur de température,
dans lequel le dispositif de commande est configuré pour estimer la durée de vie restante de l'unité de détection uniquement lorsque la valeur de température mesurée par le capteur de température est 20 à 28 degrés C.

8. Ethylomètre selon la revendication 1, dans lequel l'unité de calcul d'alcoolémie est configurée pour calculer l'alcoolémie en comparant une valeur obtenue par intégration de la valeur actuelle reçue de l'unité de détection au fil du temps à une valeur intégrale de référence.
